# EUROPEAN PATENT SPECIFICATION

(11) **EP 2 227 272 B1**
(45) Date of publication and mention of the grant of the patent: **27.02.2013**
(21) Application number: 08851354.4
(22) Date of filing: 21.11.2008
(51) Int. Cl.: A61M 3/02

(54) **CANNULA FOR DISPENSING FLUID PRODUCTS, PARTICULARLY FOR RECTAL APPLICATIONS**
KANÜLE ZUR ABGABE VON FLÜSSIGEN PRODUKTEN, INSBESONDERE FÜR REKTALE ANWENDUNGEN
CANULE DE DISTRIBUTION DE PRODUITS FLUIDES, EN PARTICULIER POUR APPLICATIONS RECTALES

(30) Priority: 23.11.2007 IT MO20070357
(43) Date of publication of application: 15.09.2010
(73) Proprietor: Lameplast S.P.A., Frazione Rovereto Sul Secchia (IT)
(72) Inventor: FONTANA, Antonio, I-41012 Carpi (MO) (IT)
(74) Representative: Brunacci, Marco
(86) International application number: PCT/IB2008/003174
(87) International publication number: WO 2009/066164

(56) References cited:
- EP-B- 0 334 349
- WO-A-2006/134464
- US-A- 4 248 228
- US-A- 5 219 448
- US-A1- 2003 088 217

## Description

### Technical Field

This invention relates to a cannula for dispensing fluid products, particularly for rectal applications.

### Background Art

Various hygienic-sanitary devices exist for the dispensing of medicinal fluid products, specifically designed for rectal applications.

One of these devices consists of traditional enemas which, in point of fact, consist of a deformable pear-shaped flexible-rubber bag that terminates with a dispenser pipe.

The enemas are filled with the medicinal product before use; following the squeezing of the flexible-rubber bag, therefore, the product to be administered is pushed inside the dispenser pipe from which it exits through an opening in the extremity.

Another device of known type is composed of plastic bottles shaped like a bellows, which also have a dispenser pipe but which, unlike the enemas, do not have a flexible-rubber bag, but a container that folds on itself like a bellows. These bottles work in the same way as the enemas, because in the case of the bottles as well, the product is dispensed through the pipe by squeezing the bellows container.

Both the enemas and the bottles, however, have various drawbacks, such as, for example, the fact that they are affected by rather high overall dimensions and inappropriate limitations as regards use.

In this respect, the fact is emphasised that to apply products in fluid state in the form of paste or cream, these devices of known type are particularly inconvenient and awkward to use and, in some cases, are totally inefficient.

Furthermore, it must not be forgotten that the dimensions and the shape of these devices do not always allow dispensing the totality of the products contained in them, which, on the contrary, remains trapped, at least in part, in the flexible-rubber bag or in the bellows container, thus representing a pointless waste.

Other devices are also known for the application of medical fluids made up of a tube, containing the product to be dispensed, and an internally hollow dispensing rod, which is initially empty and separated from the tube.

The tube has an exit opening for the fluid product which has a threaded spout onto which the dispensing rod is screwed before use.

The dispensing rod, in turn, has a series of openings obtained on its side surface. During the application phase therefore, the squeezing of the tube pushes the fluid product along the dispensing rod until it reaches its side openings, through which the fluid product is distributed directly in contact with the walls of the body cavity to be medicated.

This particular type of device does however also have several drawbacks related, in particular, to the considerable difficulties associated with use and to the impossibility, as in the case of the enemas and bottles already illustrated, to dispense all the medical product contained in the tube.

Inside the dispensing rod, in fact, at least partially trapped, remains a certain quantity of fluid to be dispensed; such quantity cannot be administered and is pointlessly wasted.

An other sanitary instrument is know from WO 2006/134464, which discloses a syringe-like device substantially similar to the previous device, with the exception that the squeezable tube containing the fluid product is substituted by a rigid hollow and cylindrical body in which a piston can slide.

The rigid body is suitable to support the dispensing rod, through which the fluid product can be dispensed under the pressure of the piston.

However, the rigid body according to WO 2006/134464 has the same drawbacks of the squeezable tube because the piston can move only inside the rigid body and not inside the dispenser rod; consequently, a part of the fluid product remains at least partially trapped inside the dispensing rod and is wasted.

Also known are cannulas which, generally, are sold in packs together with tubes or bottles containing the product to be applied.

The known cannulas are composed of a cylinder suitable for containing the product and inside which slides a piston integral with the extremity of a push rod.

The piston is usually composed of a flat-shaped plate fitted sliding along the inner side walls of the cannula.

The cylinder has an open extremity that can be coupled with the dispenser mouth of the tube, to introduce into the cylinder itself the quantity of the product to be applied, and through which the introduced product is dispensed. The opposite extremity of the cylinder is closed by a bottom which is provided with a hole, in which the push rod is fitted sliding, and which acts as a stop element for the piston to prevent this slipping out.

The product introduced into the cannula is dispensed by operating the push rod in the direction of movement of the piston towards the open extremity of the cylinder.

Another type of cannula is composed of a cylinder, which has its opposite extremities open and inside which is fitted sliding a plate-shaped piston, and of a push rod separated from the piston and which can be coupled to this in a removable way.

At the opposite extremities of the cylinder, sub-squares or stop shoulders are obtained suitable for stopping piston sliding and preventing the piston from coming out as a result of the action of the push rod.

These latter cannulas can be sold in packs containing a single push rod, a plurality of empty disposable cylinders to be used for the different applications and one or more tubes of product.

In this case one of the two extremities of the cylinders can be coupled with the dispenser mouth of the tube in order to introduce the product inside the cylinders themselves, while the opposite extremity acts as a passage for the push rod.

Alternatively, these cannulas can be sold in packs containing a single push rod and a plurality of cylinders already filled with the product to be applied and having two opposite extremities with closing caps that are removed at the time of use.

The cannulas of known type are however also liable to further targeted upgrading, in particular, to make them more practical and easier to use.

In this respect, it should be noted that the traditional cannulas, having just one dispenser opening placed axially at the free extremity of the cannula, make it possible to allow the product to be dispensed to flow in a longitudinal direction only.

This considerably restricts use considering that some medical products need to be distributed and/or spread over larger surface extensions.

Furthermore, traditional cannulas are often not easy to handle and practical to use.

It should not be forgotten moreover that traditional cannulas are sometimes very complicated to make and, therefore, involve rather high production costs that negatively affect the retail price to the public.

Other kinds of applicators are known from patent documents US 5,219,448, EP 0 334 349 and WO 2006/134464.

### Object of the Invention

The main aim of the present invention is to provide a cannula for dispensing fluid products, particularly for rectal applications, that allows achieving the above-mentioned standards of upgrading, that has compact overall dimensions and is very easy to handle and which can be simply and promptly used by users, and which is quick to manufacture, make up and package.

Another object of the present invention is to provide a cannula for dispensing fluid products, particularly for rectal applications, that allows overcoming the mentioned drawbacks of the state of the art as part of a simple and rational solution, that is easy and effective to use and of low cost.

The above objects are achieved by the present cannula for dispensing fluid products, particularly for rectal applications, according to the contents of claim 1.

### Short Description of the Drawings

Other characteristics and advantages of the present invention will become clearer from the description of some preferred but not sole embodiments of a cannula for dispensing fluid products, particularly for rectal applications, including but not limited to that shown on the attached drawing tables in which:
the figure 1 is an exploded view of a preferred embodiment of the cannula according to the invention;
the figure 2 is an axonometric, partially cross-section view of the cannula of figure 1 in the initial packaging configuration;
the figure 3 is a section view of the cannula of figure 1 in the initial packaging configuration;
the figure 4 is an axonometric, partially cross-section view, of the cannula of figure 1 in the final complete dispensing configuration;
the figure 5 is a section of the cannula of figure 1 in the final complete dispensing configuration;

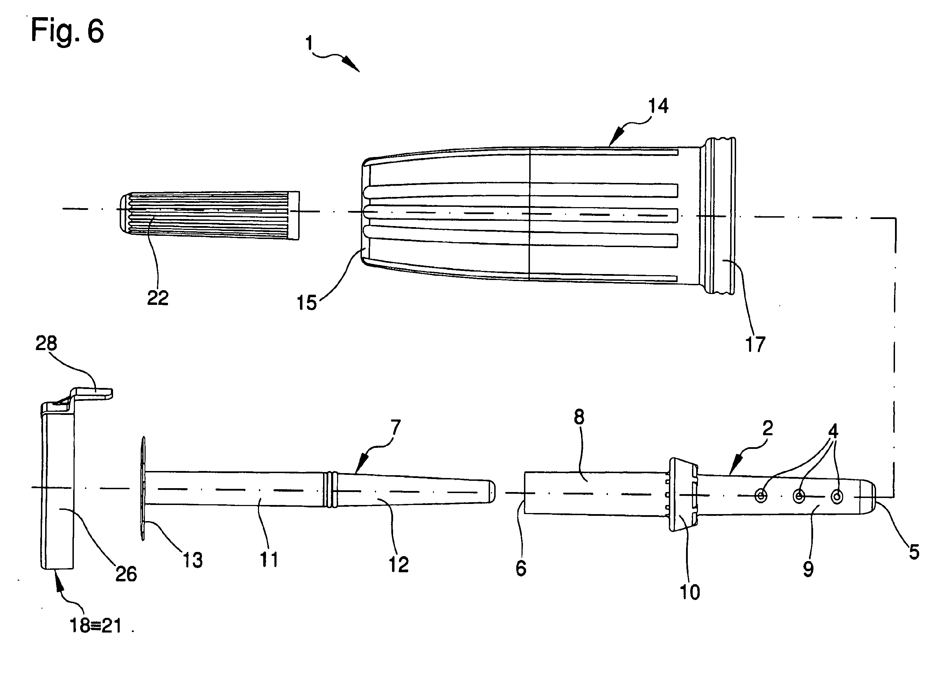

the figure 6 is an exploded view of an alternative embodiment of the cannula according to the invention;
the figure 7 is an axonometric, partially cross-section view of the cannula of the figure 6 in the initial packaging configuration;
the figure 8 is a section view of the cannula of figure 6 in the initial packaging configuration;
the figure 9 is an axonometric, partially cross-section view of the cannula of figure 6 in the final complete dispensing configuration;
the figure 10 is a section view of the cannula of figure 6 in the final complete dispensing configuration.

### Embodiments of the Invention

With particular reference to these figures, by 1 has been indicated a cannula for dispensing fluid products, particularly for rectal applications.

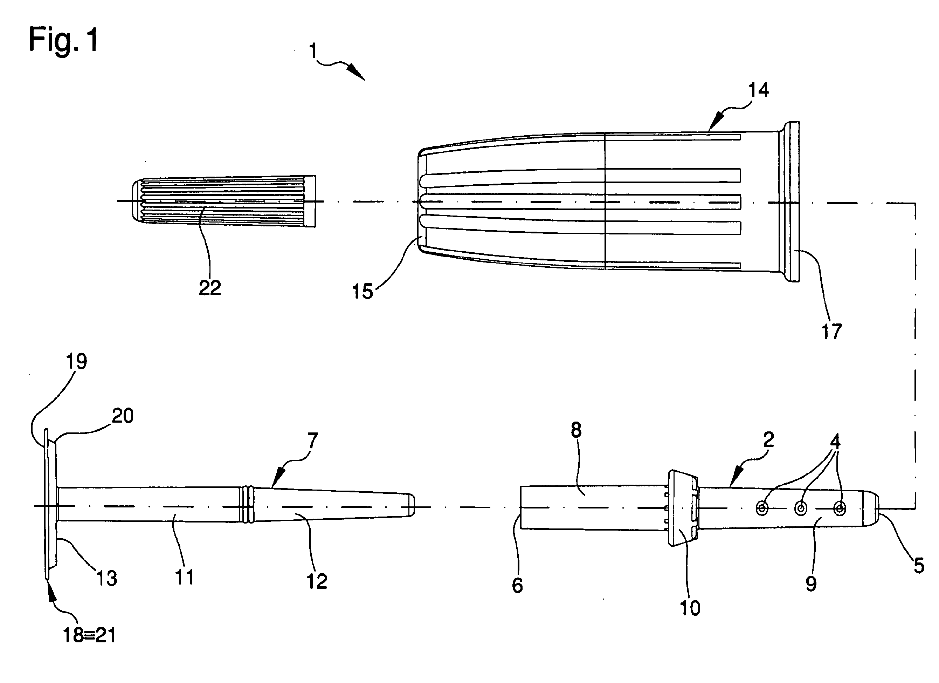

In a first embodiment of the invention illustrated in the figures 1 to 5, the cannula 1 comprises a round-section tubular body 2 suitable for containing a product 3 to be dispensed which is in fluid state and, e.g., is of the type of liquids, creams, pastes or the like.

The tubular body 2 is at least in part pre-filled, i.e., it is more or less completely filled with the product 3 during the packaging phase before the cannula 1 is distributed on the market.

The tubular body 2 has a plurality of dispensing mouths 4 for dispensing the product 3 obtained on its side surface and has a closed axial extremity 5 and an open axial extremity 6.

Through the open axial extremity 6 in the tubular body 2 a push piston 7 of the product 3 is fitted axially sliding, which push piston is moving between an initial packaging configuration and a final complete dispensing configuration. In detail, the tubular body 2 consists of a first portion 8, cylindrical in shape, that starts with the open axial extremity 6 and along which the push piston 7 slides slipping, and of a second portion 9 that protrudes from the first portion 8, ends in the closed axial extremity 5 and along which are obtained the dispensing mouths 4.

The first portion 8 and the second portion 9 are joined together at the median part 10 of the tubular body 2.

The second portion 9 has a substantially truncated-cone shape, in the sense that, near the closed axial extremity 5, it has a slightly narrower cross section than the cross section near the first portion 8.

In the same way, the push piston 7 consists of a first section 11 cylindrical in shape, that slides slipping inside the first portion 8 of the tubular body 2, and of a second section 12 with a truncated-cone shape, which protrudes from the first section 11 and is substantially elongated in the longitudinal direction of the tubular body 2.

In the final complete dispensing configuration, the second section 12 can be placed substantially in manner of mating with the inner surface of the second portion 9 of the tubular body 2.

The second section 12, in point of fact, is designed to push the product 3 through the dispensing mouths 4, moving from the initial packaging configuration to the final complete dispensing configuration.

During forward movement, the truncated-cone shaped surface of the second section 12 permits giving the product 3 both an axial push and a sideways push towards the dispensing mouths 4, allowing the substantial totality of the product 3 to come out of the tubular body 2.

At the same time, the shape of the second section 12 allows obtaining the complete occlusion of the dispensing mouths 4 once the final complete dispensing configuration has been achieved, so as to prevent any return flows of the product 3 inside the second portion 9.

Advantageously, in the initial packaging configuration, the first section 11 of the push piston 7 is partially fitted in the tubular body 2 and, in point of fact, represents its closing bottom which prevents the product 3 from coming out through the open axial extremity 6.

Furthermore, the push piston 7 comprises a round-shaped pad 13 that extends crossways from the end extremity of the first section 11 opposite the second section 12 and is suitable for representing an operating plate for the user.

With the tubular body 2 is associable a substantially tubular liner 14 which extends coaxial around the first portion of the tubular body 2 and the push piston 7.

In detail, the liner 14 has a first extremity 15, on which is mounted a connection partition 16 associable with the median part 10 of the tubular body 2, and a second extremity 17, opposite the first, which is open to allow the introduction of the push piston 7.

In the fitting configuration, the liner 14 extends from the median part 10 of the tubular body 2 well over the open axial extremity 6 of the tubular body 2; in practice, this means that, with respect to the position of the connection partition 16, the plane position of the second extremity 17 is further away than the plane position of the open axial extremity 6.

The cross dimensions of the liner 14, furthermore, are substantially complementary to the dimensions of the pad 13 and such as to allow the insertion of one or more of the user's fingers in order to operate the push piston 7.

At the second extremity 17 of the liner 14 temporary withholding means 18 are provided for withholding the push piston 7 in the initial packaging configuration which are placed between the liner 14 and the pad 13 of the push piston 7.

The temporary withholding means 18, for example, comprise an annular edge 19 which is associated with the pad 13 along a preset fracture line 20 and which is fixable inside the second extremity 17 of the liner 14.

For this purpose, the second extremity 17 of the liner 14 has an inner groove into which can be slotted the annular edge 19.

Usefully, first anti-tampering means 21 are also provided, suitable for sealing the initial packaging configuration.

The first anti-tampering means 21 are placed between the liner 14 and the push piston 7 and, in the particular embodiment of the invention shown in the illustrations from 1 to 5, coincide with the temporary withholding means 18. The integrity of the preset fracture line 20 and the presence of the annular edge 19 attached to the pad 13, in fact, testify to the perfect preservation of the cannula 1, proving that this has not been tampered with after fabrication and that the product 3 inside it has not been unnaturally damaged.

Alternative embodiments cannot however be ruled out in which the first anti-tampering means 21 are different and distinct from the temporary withholding means 18.

Advantageously, the dispensing mouths 4 are round shaped and split up into a first series distributed longitudinally on the tubular body 2 and into a second series distributed longitudinally diametrically opposite the first series. Alternative embodiments are however possible in which there is only one dispensing mouth 4 which, for example, is elongated in the longitudinal direction of the tubular body 2 or has a helical shape that wraps around the second portion 9 like a spiral.

The shape, the dimensions and the position of the dispensing mouths 4 along the tubular body 2 can be differentiated according to the type of product 3 used and to the pharmaceutical treatment to be applied.

In this way, in fact, a distribution of the product 3 can be obtained on a more or less ample surface and in a more or less uniform and/or localised way.

To temporarily close the dispensing mouths 4 a cap 22 is provided that can be snugly fitted around the second portion 9 of the tubular body 2.

In between the cap 22 and the connection partition 16 are placed second anti-tampering means 23, suitable for sealing the closure of the dispensing mouths 4 and underlining the separation of the cap 22 from the tubular body 2.

The second anti-tampering means 23 consist for example of a breakable seal line placed between the open edge of the cap 22 and the connection partition 16. Alternative embodiments cannot however be ruled out in which the cap 22 is temporarily sealable on the tubular body 2 instead of on the connection partition 16.

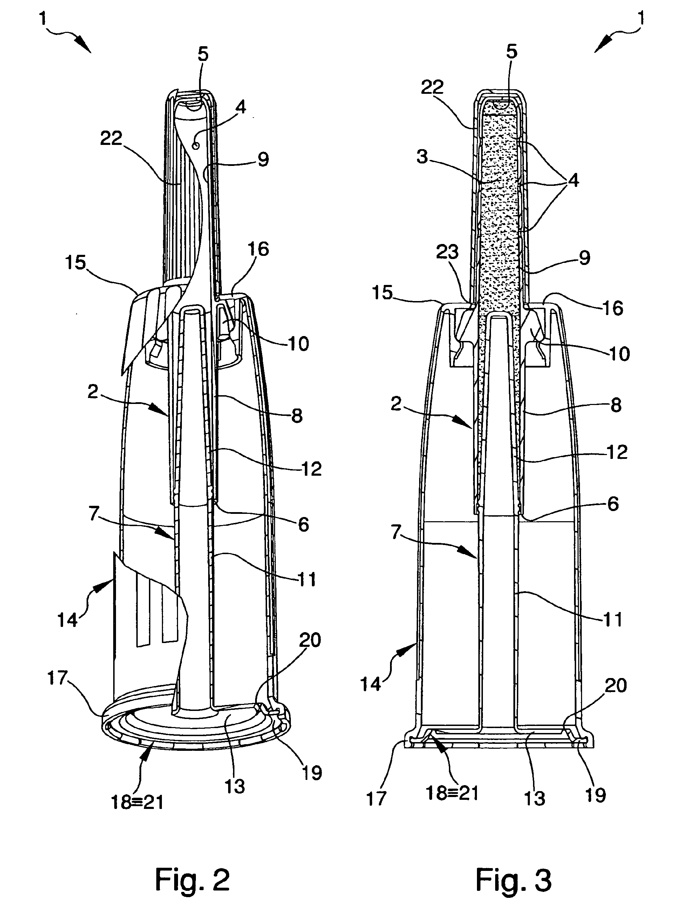

The cannula 1 is sold in the initial packaging configuration (figures 2 and 3) and is pre-filled with the product 3; in this configuration, the annular edge 19 is attached to the pad 13 while the cap 22 is fitted on the second portion 9 and is temporarily secured, by means of the breakable seal line 23, to the connection partition 16.

To use the cannula 1 proceed to break the breakable seal line 23 and remove the cap 22 from the tubular body 2.

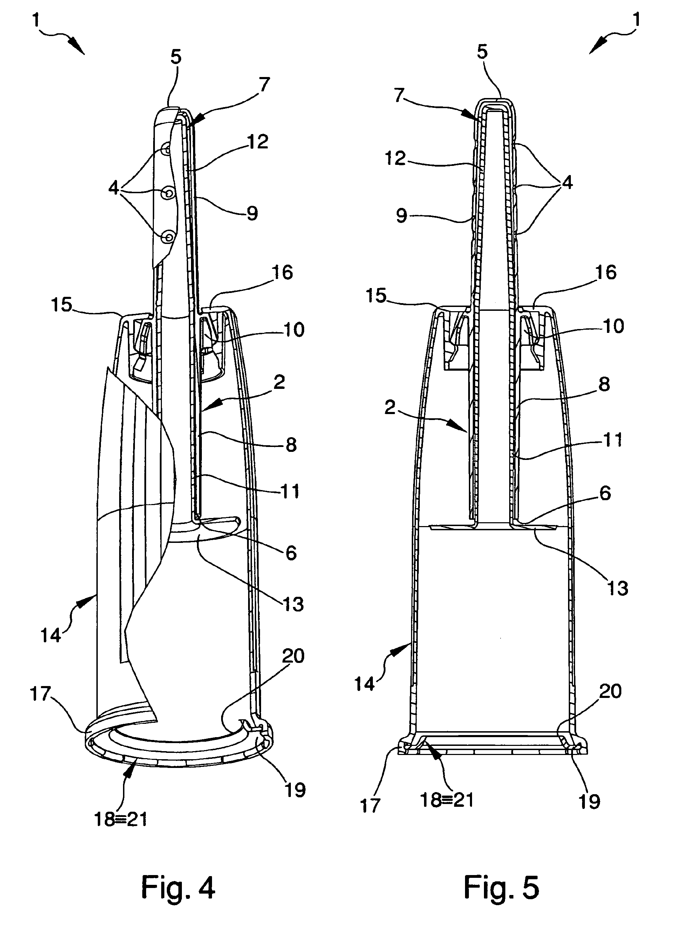

At this point, the application of a push force on the pad 13 by the user allows breaking the preset fracture line 20 and freeing the push piston 7, which can thus be made to slide as far as the complete dispensing configuration (figures 4 and 5) to eject the product 3 through the dispensing mouths 4.

In an alternative embodiment of the invention shown in the figures from 6 to 10, the cannula 1 has a tubular body 2, a push piston 7, a liner 14 and a cap 22 substantially the same as those of the previously illustrated embodiment.

In this form of embodiment however the temporary withholding means 18 are much different and comprise an annular-shaped protrusion 24 obtained inside the second extremity 17 of the liner 14.

The protrusion 24 is suitable for acting as a shoulder for the pad 13 and preventing the sliding of the push piston 7 towards the inside of the tubular body 2 without the user applying any preset force.

The temporary withholding means 18, furthermore, comprise a closing wall 25 that temporarily closes the second extremity 17 of the liner 14.

The closing wall 25 is suitable for preventing the sliding of the push piston 7 towards the outside of the tubular body 2.

In detail, the closing wall 25 is associable removable with the liner 14 by means of a strip 26, which is wrapped around the second extremity 17 and which supports the closing wall 25 by interposition of a weakened-section edge 27. The strip 26 is of the tear-off type and has a grip and tear-off flap 28 suitable for breaking the strip 26 to remove it from the liner 14.

During the removal of the strip 26, the weakened-section edge 27 is suitable for breaking, leaving the piston 7 free to slide inside the tubular body 2.

Usefully, also in this embodiment of the invention the first anti-tampering means 21 are provided, which are suitable for sealing the initial packaging configuration and coinciding with the temporary withholding means 18.

The presence of the strip 26 attached to the liner 14, does in fact show the integrity of the cannula 1, proving that this has not been tampered with after fabrication and that the product 3 has not been damaged in any way inside.

In the embodiment shown in the figures from 6 to 10, furthermore, the liner 14 has guide means 29 for the sliding of the push piston 7.

Such guide means comprise a plurality of longitudinal ridges arranged on the inner surface of the liner 14 on which the edge of the pad 13 slides slipping. The longitudinal ridges 29, besides guiding the sliding of the pad 13, also allow reinforcing the liner 14 increasing its strength and overall sturdiness.

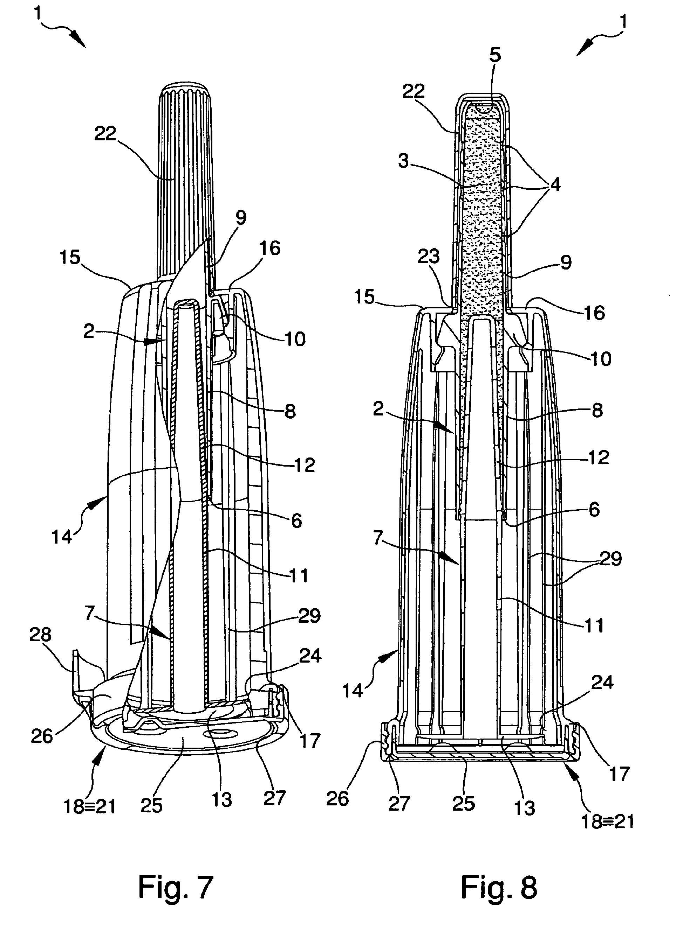

The cannula 1 according to the embodiment of the figures from 6 to 10 is sold in the initial packaging configuration (figures 7 and 8) filled with the product 3; in this configuration the strip 26 is fitted on the liner 14 and is associated with the closing wall 25, while the cap 22 is fitted on the second portion 9 of the tubular body 2 and is temporarily fastened, by means of the breakable seal line 23 to the connection partition 16.

To use the cannula 1 the grip and tear-off flap 28 must be torn off, the strip 26 and the closing wall 25 removed, the tearing line 19 broken and the cap 22 removed from the tubular body 2.

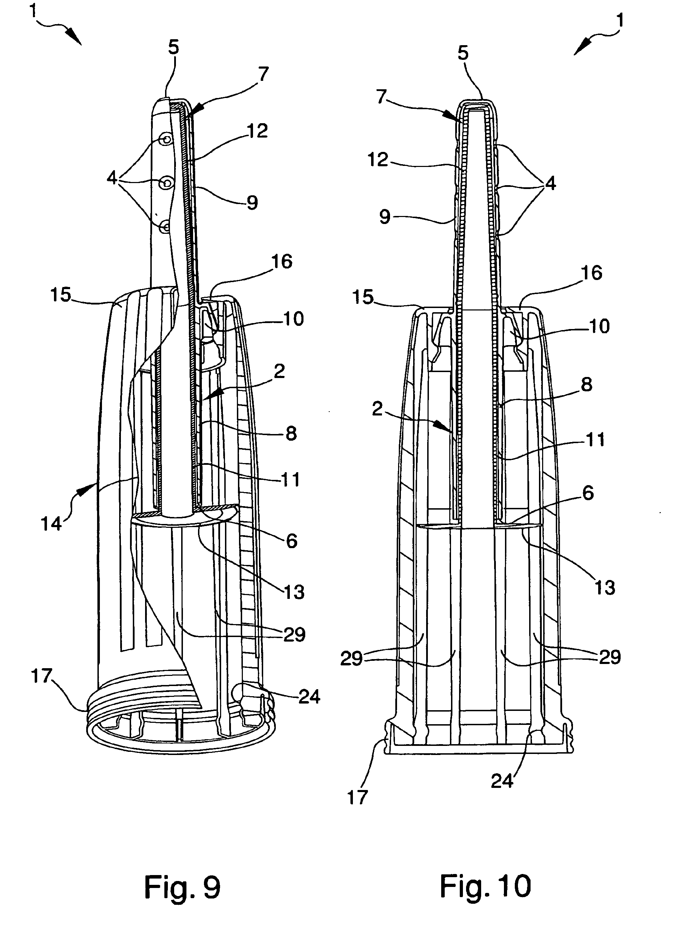

At this point, the cannula 1 is ready for application, which can be done by the simple manual action of the user, by pushing on the pad 13 to overcome the obstacle represented by the protrusion 24, moving the piston 7 as far as the complete dispensing configuration (figures 9 and 10) and expelling in this way the product 3 through the dispensing mouths 4.

It has in practice been demonstrated how the described invention achieves the proposed objects.

In this respect, the fact is underlined that the particular solution of providing dispensing mouths arranged on the side surface of the cannula permits the more practical and successful distribution of the product to be dispensed by means of a very simple, compact and inexpensive cannula.

It must also be reiterated that the particular truncated-cone shape of the piston makes possible easier side exit of the product to be dispensed, permits fully emptying the tubular body and prevents any product back flows inside the cannula.

It should not be forgotten on the other hand that the presence of the liner associated with the tubular body allows protecting the push piston in safety conditions, assuring the preservation of the cannula in the initial packaging configuration and avoiding any risk of the push piston undergoing damage such as to negatively affect normal operation.

The invention thus conceived is susceptible to numerous modifications and variations, all of which falling within the scope of the inventive concept. Furthermore all the details can be replaced with others that are technically equivalent.

In practice, the materials used, as well as the contingent shapes and dimensions, may be any according to requirements without because of this moving outside the protection scope of the following claims.

## Claims

1. Cannula (1) for dispensing fluid products, particularly for rectal applications, comprising:
- at least a tubular body (2) for containing a fluid product (3) to be dispensed which has at least a product dispensing mouth (4) and at least an open axial extremity (6), said dispensing mouth (4) being obtained on the side surface of said tubular body (2),
- at least a cap (22) fittable substantially snugly around said tubular body (2) for temporarily closing said dispensing mouth (4),
- at least a push piston (7) for pushing said product (3) which is at least partially fittable in said tubular body (2) through said open axial extremity (6) and moving between an initial packaging configuration and a final dispensing configuration,
- at least a substantially tubular liner (14) which is associable with said tubular body (2) around said push piston (7),
- and temporary withholding means (18) for withholding said push piston (7) in said initial packaging configuration which are placed in between said liner (14) and said push piston (7),
**characterized by** the fact that it comprises at least one between:
- first anti-tampering means (21) for sealing said initial packaging configuration coinciding with said temporary withholding means (18), and
- second anti-tampering means (23) for sealing the fastening of said dispensing mouth (4), which are suitable for underlining the separation of said cap (22) from said tubular body (2).

2. Cannula (1) according to claim 1, **characterized in that** it comprises a plurality of said dispensing mouths (4).

3. Cannula (1) according to claim 2, **characterized in that** said dispensing mouths (4) are split up into at least a first series distributed longitudinally on said tubular body (2).

4. Cannula (1) according to one or more of the previous claims, **characterized in that** said tubular body (2) comprises at least a first portion (8), with a substantially cylindrical shape, along which said push piston (7) slides slipping.

5. Cannula (1) according to claim 4, **characterized in that** said tubular body (2) comprises at least a second portion (9), with a substantially truncated-cone shape, which extends from said first portion (8) and along which said dispensing mouth (4) is obtained.

6. Cannula (1) according to one or more of the previous claims, **characterized in that** said push piston (7) comprises at least a first section (11), with a substantially cylindrical shape, that slides slipping inside said first portion (8) of the tubular body (2).

7. Cannula (1) according to claim 6, **characterized in that** said push piston (7) comprises at least a second section (12), with a substantially truncated-cone shape, which protrudes longitudinally from said first section (11) and, in the final dispensing configuration, can be placed substantially in manner of mating with the inner surface of said second portion (9) of the tubular body (2).

8. Cannula (1) according to claim 6 or 7, **characterized in that** said push piston (7) comprises at least a pad (13) that extends crossways from the end extremity of said first section (11).

9. Cannula (1) according to one or more of the previous claims, **characterized in that** said liner (14) comprises at least a first extremity (15) associated with a connection partition (16) to said tubular body (2), and at least a second extremity (17), opposite the first, which is substantially open.

10. Cannula (1) according to claim 9, **characterized in that** said connection partition (16) is associable with the substantially median part (10) of said tubular body (2).

11. Cannula (1) according to one or more of the previous claims, **characterized in that** said liner (14) extends substantially well over said open axial extremity (6) of the tubular body (2).

12. Cannula (1) according to one or more of the previous claims, **characterized in that** said liner (14) comprises guide means (29) for the sliding of said push piston (7) which comprise a plurality of longitudinal ridges arranged on the inner surface of said liner (14) on which the edge of said pad (13) slides slipping.

13. Cannula (1) according to one or more of the previous claims, **characterized in that** said temporary withholding means (18) comprise at least an annular edge (19) which is associated with said push piston (7) along a preset fracture line (20) and which is fixable to said liner (14).

14. Cannula (1) according to claim 13, **characterized in that** said annular edge (19) is fixable inside said second extremity (17) of the liner (14).

15. Cannula (1) according to one or more of the previous claims, **characterized in that** said second extremity (17) of the liner (14) has at least an inner groove into which can be slotted said annular edge (19).

16. Cannula (1) according to one or more of the previous claims, **characterized in that** said temporary withholding means (18) comprise at least a protrusion (24) obtained inside said liner (14) and suitable for preventing the sliding of said push piston (7) towards the inside of said tubular body (2) without applying any preset force.

17. Cannula (1) according to one or more of the previous claims, **characterized in that** said temporary withholding means (18) comprise at least a closing wall (25) of said second extremity (17) of the liner (14) which is suitable for preventing the sliding of said push piston (7) towards the outside of said tubular body (2) and which closing wall (25) is associable removable with said liner (14).

18. Cannula (1) according to one or more of the previous claims, **characterized in that** said temporary withholding means (18) comprise at least a strip (26) wrapped around said second extremity (17) of the liner (14) and associated with said closing wall (25) along a weakened-section edge (27).

19. Cannula (1) according to claim 18, **characterized in that** said strip (26) comprises at least a grip and tear-off flap (28) suitable for breaking said strip (26) to remove it from said liner (14).

20. Cannula (1) according to one or more of the previous claims, **characterized in that** said second anti-tampering means (23) comprise at least a breakable seal line placed between the open edge of said cap (22) and at least one between said tubular body (2) and said liner (14).

21. Cannula (1) according to one or more of the previous claims, **characterized in that** said tubular body (2) is at least in part pre-filled with said fluid product (3).

## Patentansprüche

1. Kanüle (1) zur Abgabe von flüssigen Produkten, insbesondere für rektale Anwendungen, enthaltend:
- mindestens einen rohrförmigen Körper (2) zur Aufnahme eines abzugebenden flüssigen Produkts (3), welcher mindestens eine Produktabgabeöffnung (4) und mindestens ein offenes axiales Ende (6) hat, welche Abgabeöffnung (4) auf der Seitenfläche des rohrförmigen Körpers (2) angeordnet ist,
- mindestens eine Kappe (22), die im Wesentlichen mit engem Sitz auf den rohrförmigen Körper (2) zum zeitweiligen Verschließen der Abgabeöffnung (4) aufsetzbar ist,
- mindestens einen Druckkolben (7), um das Produkt (3) auszudrücken, welcher wenigstens teilweise in den rohrförmigen Körper (2) durch das offene axiale Ende (6) einsetzbar ist und sich zwischen einer ursprünglichen Verpackungskonfiguration und einer endgültigen Abgabekonfiguration bewegt,
- mindestens eine im Wesentlichen rohrförmige Umhüllung (14), die mit dem rohrförmigen Körper (2) um den Druckkolben (7) verbunden werden kann,
- und zeitweilige Rückhaltemittel (18), um den Druckkolben (7) in der ursprünglichen Verpackungskonfiguration zurückzuhalten, welche zwischen der Umhüllung (14) und dem Druckkolben (7) angeordnet sind,
**gekennzeichnet durch** die Tatsache, dass sie mindestens eines der folgenden Mittel aufweist:
- ein erstes Missbrauchsschutzmittel (21) zum Versiegeln der ursprünglichen Verpackungskonfiguration, welches sich mit den zeitweiligen Rückhaltemitteln deckt, und
- ein zweites Missbrauchsschutzmittel (23) zum Versiegeln des Verschlusses der Ausgabeöffnung (4), die zur Unterlegung der Trennstelle der Kappe (22) von dem rohrförmigen Körper (2) geeignet sind.

2. Kanüle (1) nach Anspruch 1, **dadurch gekennzeichnet, dass** sie eine Vielzahl der Ausgabeöffnungen (4) aufweist.

3. Kanüle (1) nach Anspruch 2, **dadurch gekennzeichnet, dass** die Ausgabeöffnungen (4) in mindestens eine erste Reihe aufgeteilt sind, die in Längsrichtung auf dem rohrförmigen Körper (2) verteilt ist.

4. Kanüle (1) nach einem oder mehreren der vorhergehenden Ansprüche, **dadurch gekennzeichnet, dass** der rohrförmige Körper (2) mindestens einen ersten Abschnitt (8) mit einer im Wesentlichen zylindrischen Form aufweist, entlang welchem der Druckkolben (7) verschieblich gleitet.

5. Kanüle (1) nach Anspruch 4, **dadurch gekennzeichnet, dass** der rohrförmige Körper (2) mindestens einen zweiten Abschnitt (9) mit einer im Wesentlichen kegelstumpfförmigen Form aufweist, welche sich von dem ersten Abschnitt (8) erstreckt und entlang welchem die Ausgabeöffnung (4) angeordnet ist.

6. Kanüle (1) nach einem oder mehreren der vorhergehenden Ansprüche, **dadurch gekennzeichnet, dass** der Druckkolben (7) mindestens einen ersten Abschnitt (11) mit einer im Wesentlichen zylindrischen Form aufweist, der innerhalb des ersten Abschnitts (8) des rohrförmigen Körpers (2) verschieblich gleitet.

7. Kanüle (1) nach Anspruch 6, **dadurch gekennzeichnet, dass** der Druckkolben (7) mindestens einen zweiten Abschnitt (12) mit einer im Wesentlichen kegelstumpfförmigen Form aufweist, welcher in Längsrichtung von dem ersten Abschnitt (11) vorspringt und in der endgültigen Ausgabekonfiguration im Wesentlichen in zusammenpassender Weise mit der Innenfläche des zweiten Abschnitts (9) des rohrförmigen Körpers (2) positioniert werden kann.

8. Kanüle (1) nach Anspruch 6 oder 7, **dadurch gekennzeichnet, dass** der Druckkolben (7) mindestens eine Platte (13) aufweist, die sich in Querrichtung von dem Endabschnitt des ersten Abschnitts (11) erstreckt.

9. Kanüle (1) nach einem oder mehreren der vorhergehenden Ansprüche, **dadurch gekennzeichnet, dass** die Umhüllung (14) mindestens ein erstes Ende (15) aufweist, das mit einer Verbindungszwischenwand (16) mit dem rohrförmigen Körper (2) verbunden ist, sowie mindestens ein zweites, dem ersten Ende entgegengesetztes Ende (17), welches im Wesentlichen offen ist.

10. Kanüle (1) nach Anspruch 9, **dadurch gekennzeichnet, dass** die Verbindungszwischenwand (16) mit dem im Wesentlichen mittleren Teil (10) des rohrförmigen Körpers (2) verbindbar ist.

11. Kanüle (1) nach einem oder mehreren der vorhergehenden Ansprüche, **dadurch gekennzeichnet, dass** die Umhüllung (14) sich im Wesentlichen deutlich über das offene axiale Ende (6) des rohrförmigen Körpers (2) hinaus erstreckt.

12. Kanüle (1) nach einem oder mehreren der vorhergehenden Ansprüche, **dadurch gekennzeichnet, dass** die Umhüllung (14) Führungsmittel (29) für das Verschieben des Druckkolbens (7) aufweist, welche eine Vielzahl von Längsrippen umfassen, die an der inneren Oberfläche der Umhüllung (14) angeordnet sind, auf welchen der Rand der Platte (13) verschiebbar gleitet.

13. Kanüle (1) nach einem oder mehreren der vorhergehenden Ansprüche, **dadurch gekennzeichnet, dass** das zeitweilige Rückhaltemittel (18) mindestens einen ringförmigen Rand (19) aufweist, welcher mit dem Druckkolben (7) entlang einer voreingestellten Bruchlinie (20) verbunden ist und der an der Umhüllung (14) befestigtbar ist.

14. Kanüle (1) nach Anspruch 13, **dadurch gekennzeichnet, dass** der ringförmige Rand (19) innerhalb des zweiten Endes (17) der Umhüllung (14) fixierbar ist.

15. Kanüle (1) nach einem oder mehreren der vorhergehenden Ansprüche, **dadurch gekennzeichnet, dass** das zweite Ende (17) der Umhüllung (14) mindestens eine innere Nut hat, in welche der ringförmige Rand (19) eingesetzt werden kann.

16. Kanüle (1) nach einem oder mehreren der vorhergehenden Ansprüche, **dadurch gekennzeichnet, dass** das zeitweilige Rückhaltemittel (18) mindestens einen Vorsprung (24) aufweist, der innerhalb der Umhüllung (14) vorgesehen ist und dafür geeignet ist, das Verschieben des Druckkolbens (7) zur Innenseite des rohrförmigen Körpers (2) hin ohne Anwendung einer voreingestellten Kraft zu verhindern.

17. Kanüle (1) nach einem oder mehreren der vorhergehenden Ansprüche, **dadurch gekennzeichnet, dass** die zeitweilige Rückhalteeinrichtung (18) mindestens eine Verschlusswand (25) des zweiten Endes (17) der Umhüllung (14) umfasst, die dazu geeignet ist, das Verschieben des Druckkolbens (7) zur Außenseite des rohrförmigen Körpers (2) zu verhindern, welche Verschlusswand (25) lösbar mit der Umhüllung (14) verbindbar ist.

18. Kanüle (1) nach einem oder mehreren der vorhergehenden Ansprüche, **dadurch gekennzeichnet, dass** die zeitweilige Rückhalteeinrichtung (18) mindestens einen Streifen (26) aufweist, der um das zweite Ende (17) der Umhüllung (14) geschlungen ist und mit der Verschlusswand (25) entlang einem Rand (27) mit einem geschwächten Abschnitt verbunden ist.

19. Kanüle (1) nach Anspruch 18, **dadurch gekennzeichnet, dass** der Streifen (26) mindestens eine Greif- und Abreißlasche (28) aufweist, die dafür geeignet ist, den Streifen (26) aufzubrechen, um ihn von der Umhüllung (14) zu entfernen.

20. Kanüle (1) nach einem oder mehreren der vorhergehenden Ansprüche, **dadurch gekennzeichnet, dass** die zweite Missbrauchsschutzeinrichtung (23) mindestens eine aufbrechbare Versiegelungslinie aufweist, die zwischen dem offenen Rand der Kappe (23) und mindestens entweder zwischen dem rohrförmigen Körper (2) oder der Umhüllung (14) oder beidem angeordnet ist.

21. Kanüle (1) nach einem oder mehreren der vorhergehenden Ansprüche, **dadurch gekennzeichnet, dass** der rohrförmige Körper (2) zumindest teilweise mit dem flüssigen Produkt (3) vorgefüllt ist.

## Revendications

1. Canule (1) pour délivrer des produits fluides destinés à des applications rectales, comprenant :
- au moins un corps tubulaire (2) destiné à contenir un produit fluide (3) à distribuer, corps qui possède au moins une ouverture (4) de distribution du produit et au moins une extrémité axiale ouverte (6), ladite ouverture (4) de distribution étant ménagée sur la surface latérale dudit corps tubulaire (2),
- au moins un capuchon (22) insérable avec un ajustement essentiellement serré autour dudit corps tubulaire (2) pour fermer temporairement ladite ouverture (4) de distribution,
- au moins un piston propulseur (7) pour pousser ledit produit (3), piston qui est au moins partiellement insérable dans ledit corps tubulaire (2) par ladite extrémité axiale ouverte (6) et qui se déplace entre une configuration initiale de conditionnement et une configuration finale de distribution,
- au moins une chemise essentiellement tubulaire (14) qui est associable audit corps tubulaire (2) autour dudit piston propulseur (7),
- et des moyens de retenue temporaire (18), destinés à maintenir ledit piston propulseur (7) dans ladite configuration initiale de conditionnement, qui sont placés entre ladite chemise (14) et ledit piston propulseur (7),
***caractérisée par le fait qu'**elle* comprend au moins l'un d'entre :
- des premiers moyens d'inviolabilité (21) pour sceller ladite configuration de conditionnement initiale coïncidant avec lesdits moyens de retenue temporaire (18), et
- des seconds moyens d'inviolabilité (23) pour sceller la fermeture de ladite ouverture (4) de distribution, qui sont aptes à mettre en évidence la séparation dudit capuchon (22) d'avec ledit corps tubulaire (2).

2. Canule (1) selon la revendication 1, ***caractérisée en ce qu'**elle* comprend une pluralité desdites ouvertures (4) de distribution.

3. Canule (1) selon la revendication 2, ***caractérisée en ce que*** lesdites ouvertures (4) de distribution sont scindées en au moins une première série répartie longitudinalement sur ledit corps tubulaire (2).

4. Canule (1) selon l'une ou plusieurs des revendications précédentes, ***caractérisée en ce que*** ledit corps tubulaire (2) comprend au moins une première portion (8), de forme essentiellement cylindrique, le long de laquelle coulisse ledit piston propulseur (7).

5. Canule (1) selon la revendication 4, ***caractérisée en ce que*** ledit corps tubulaire (2) comprend au moins une deuxième portion (9), de forme essentiellement tronconique, qui part de ladite première portion (8), et le long de laquelle ladite ouverture (4) de distribution est ménagée.

6. Canule (1) selon l'une ou plusieurs des revendications précédentes, ***caractérisée en ce que*** ledit piston propulseur (7) comprend au moins une première section (11), de forme essentiellement cylindrique, qui coulisse à l'intérieur de ladite première portion (8) du corps tubulaire (2).

7. Canule (1) selon la revendication 6, ***caractérisée en ce que*** ledit piston propulseur (7) comprend au moins une deuxième section (12), de forme essentiellement tronconique, qui fait saillie longitudinalement de ladite première section (11) et, dans la configuration finale de distribution, peut être placée essentiellement en conjugaison avec la surface intérieure de ladite seconde portion (9) du corps tubulaire (2).

8. Canule (1) selon la revendication 6 ou 7, ***caractérisée en ce que*** ledit piston propulseur (7) comprend au moins une semelle (13) qui s'étend transversalement depuis l'extrémité terminale de ladite première section (11).

9. Canule (1) selon l'une ou plusieurs des revendications précédentes, ***caractérisée en ce que*** ladite chemise (14) comprend au moins une première extrémité (15), associée par une cloison de connexion (16) audit corps tubulaire (2), et au moins une deuxième extrémité (17), opposée à la première, qui est essentiellement ouverte.

10. Canule (1) selon la revendication 9, ***caractérisée en ce que*** ladite paroi de connexion (16) est associable à la partie sensiblement médiane (10) dudit corps tubulaire (2).

11. Canule (1) selon l'une ou plusieurs des revendications précédentes, ***caractérisée en ce que*** ladite chemise (14) s'étend essentiellement bien au-delà de ladite extrémité axiale ouverte (6) du corps tubulaire (2).

12. Canule (1) selon l'une ou plusieurs des revendications précédentes, ***caractérisée en ce que*** ladite chemise (14) comprend des moyens de guidage (29) pour le coulissement dudit piston propulseur (7) qui comprennent une pluralité d'arêtes longitudinales situées sur la surface intérieure de ladite chemise (14) sur lesquelles se déplace en glissant le bord de ladite semelle (13).

13. Canule (1) selon l'une ou plusieurs des revendications précédentes, ***caractérisée en ce que*** lesdits moyens de retenue temporaire (18) comprennent au moins un bord annulaire (19) qui est associé audit piston propulseur (7) le long d'une ligne pré-fracturée (20) et qui peut être fixé à ladite chemise (14).

14. Canule (1) selon la revendication 13, ***caractérisée en ce que*** ledit bord annulaire (19) peut être fixé à l'intérieur de ladite deuxième extrémité (17) de la chemise (14).

15. Canule (1) selon l'une ou plusieurs des revendications précédentes, ***caractérisée en ce que*** ladite deuxième extrémité (17) de la chemise (14) présente au moins une rainure intérieure dans laquelle peut être inséré ledit bord annulaire (19).

16. Canule (1) selon l'une ou plusieurs des revendications précédentes, ***caractérisée en ce que*** lesdits moyens de retenue temporaire (18) comprennent au moins une saillie (24) ménagée à l'intérieur de ladite chemise (14) et apte à empêcher le coulissement dudit piston propulseur (7) vers l'intérieur dudit corps tubulaire (2) sans l'application d'une force prédéterminée.

17. Canule (1) selon l'une ou plusieurs des revendications précédentes, ***caractérisée en ce que*** lesdits moyens de retenue temporaire (18) comprennent au moins une paroi de fermeture (25) de ladite deuxième extrémité (17) de la chemise (14) qui est apte à empêcher le coulissement dudit piston propulseur (7) vers l'extérieur dudit corps tubulaire (2), laquelle paroi de fermeture (25) est associable de manière amovible à ladite chemise (14).

18. Canule (1) selon l'une ou plusieurs des revendications précédentes, ***caractérisée en ce que*** lesdits moyens de retenue temporaire (18) comprennent au moins une bande (26) enroulée autour de ladite deuxième extrémité (17) de la chemise (14) et associée à ladite paroi de fermeture (25) le long d'un bord (27) à section fragilisée.

19. Canule (1) selon la revendication 18, ***caractérisée en ce que*** ladite bande (26) comprend au moins un rabat (28) de prise et déchirable apte à rompre ladite bande (26) pour la retirer de ladite chemise (14).

20. Canule (1) selon l'une ou plusieurs des revendications précédentes, ***caractérisée en ce que*** lesdits seconds moyens d'inviolabilité (23) comprennent au moins une ligne de soudure déchirable placée entre le bord ouvert dudit capuchon (22) et au moins l'un d'entre ledit corps tubulaire (2) et ladite chemise (14).

21. Canule (1) selon l'une ou plusieurs des revendications précédentes, ***caractérisée en ce que*** ledit corps tubulaire (2) est, au moins en partie, pré-rempli avec ledit produit fluide (3).
